# EUROPÄISCHE PATENTSCHRIFT

(11) **EP 3 365 318 B1**
(45) Veröffentlichungstag und Bekanntmachung des Hinweises auf die Patenterteilung: **06.01.2021**
(21) Anmeldenummer: 16778204.4
(22) Anmeldetag: 05.10.2016
(51) Int. Cl.: C07C 49/84, C07C 225/22, C07D 303/28, C07D 303/36, G02F 1/1339

(54) **BENZILMONOKETALE UND DEREN VERWENDUNG**
BENZYL MONOKETALS AND USE THEREOF
MONOCÉTALS DE BENZILE ET LEUR UTILISATION

(30) Priorität: 23.10.2015 DE 102015013754
(43) Veröffentlichungstag der Anmeldung: 29.08.2018
(73) Patentinhaber: Merck Patent GmbH, 64293 Darmstadt (DE)
(72) Erfinder: KIRSCH, Peer, 64342 Seeheim-Jugenheim (DE); TONG, Qiong, 64289 Darmstadt (DE); HAHN, Alexander, 64584 Biebesheim (DE); WEEGELS, Leo, 64297 Darmstadt (DE); GNAUCK, Steffen, 64291 Darmstadt (DE); SCHUEPFER, Sven, 63741 Aschaffenburg (DE); LEONHARD, Peter, 64291 Darmstadt (DE); ENDERS, Claudia, 64283 Gross-Umstadt (DE)
(86) Internationale Anmeldenummer: PCT/EP2016/001648
(87) Internationale Veröffentlichungsnummer: WO 2017/067634

(56) Entgegenhaltungen:
- EP-A1- 1 975 214
- WO-A1-2014/148290
- US-A1- 2009 147 206

## Beschreibung

Die vorliegende Erfindung betrifft die Verwendung hydrophiler Benzilmonoketale als Photoinitiatoren in wärme- und lichthärtenden Dichtmitteln für Flüssigkristallanzeigen, die Verwendung dieser Dichtmittel in einem Tropfverfahren (engl. One Drop Filling, ODF) zur Herstellung von Flüssigkristallanzeigen, Dichtmittel, die die hydrophilen Photoinitiatoren enthalten, sowie die mit diesen Dichtmitteln hergestellten Flüssigkristallanzeigen.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete waren anfänglich insbesondere Anzeigen für Uhren und Taschenrechner, bevor die ersten Anzeigen für beispielsweise tragbare und stationäre Computer aufkamen.

Die zunehmende Popularität der Flüssigkristallanzeigevorrichtungen (engl. Liquid Crystal Display, LCD) führte zu einer rasanten technischen Fortentwicklung und ist insbesondere darauf zurückzuführen, daß LCD gegenüber der zuvor vorherrschenden Technologie der Kathodenstrahlröhren eine geringe Leistungsaufnahme, eine leichte Bauweise und eine geringe Dicke aufweisen und keine schädliche elektromagnetische Strahlung emittieren. LCD-Vorrichtungen haben große Aufmerksamkeit auch wegen der Verwendung als großflächige Anzeigen für u.a. Fernsehgeräte erlangt, zunächst zusammen mit Plasma-Anzeigetafeln (engl.: Plasma Display Panel, PDP), mit Bildschirmgrößen von bis zu 50 Zoll und mehr.

Die LCD-Vorrichtung selbst umfaßt zwei Substrate mit Elektroden und eine in dem von den Substraten umschlossenen Raum befindliche Schicht des Flüssigkristalls. Die Anzeige eines Bildes wird durch Änderung der Ausrichtung der Flüssigkristalle mithilfe einer an die Elektroden angelegten elektrischen Spannung erreicht.

Eine LCD-Anzeige wird typischerweise hergestellt, indem ein erstes Substrat mit einer Pixelelektrode, einem Dünnfilmtransistor (engl. thin film transistor, TFT) und anderen Komponenten mit einem zweiten Substrat, das eine gemeinsame Elektrode enthält, unter Verwendung eines Dichtmittels (Versiegelungsmittels) verklebt wird. Das Befüllen des von den Substraten umschlossenen Raumes mit dem Flüssigkristall erfolgt über eine Füllöffnung durch Kapillarkraft oder Vakuum; die Füllöffnung wird anschließend unter Verwendung eines Dichtmittels versiegelt.

Mit der Zunahme der Größe der Flüssigkristallanzeigen in den letzten Jahren wurde das sogenannte "one drop filling"-Verfahren (ODF-Verfahren) vorgeschlagen als ein Verfahren zur Massenproduktion von Flüssigkristallanzeigen (siehe z.B. JPS63-179323 und JPH10-239694), um die Taktzeiten bei der Herstellung zu verkürzen. Dabei handelt es sich um ein Verfahren zur Herstellung einer Flüssigkristallanzeige, bei dem der Flüssigkristall auf ein mit Elektroden ausgestattetes Substrat getropft wird, welches von einem Dichtmittel umrandet ist. Anschließend wird das zweite mit Elektroden ausgestattete Substrat unter Vakuum montiert, das Dichtmittel durch UV-Bestrahlung teilweise gehärtet und anschließend durch Wärmebehandlung vollständig gehärtet. Durch den zweistufigen Prozeß bestehend aus UV-Härtung und thermischer Härtung kann die Dauer des Aushärtens verkürzt werden.

Dieses Verfahren hat allerdings den Nachteil, daß das Dichtmittel in ungehärtetem Zustand in Kontakt mit dem Flüssigkristall kommt und die Inhaltsstoffe des Dichtmittels in den Flüssigkristall diffundieren können.

Beim ODF-Prozeß werden drei Verfahren unterschieden:
ein Wärmehärtungsverfahren, ein Lichthärtungsverfahren und ein Licht-und Wärmehärtungsverfahren zum Härten eines Dichtmittels für Flüssigkristalle nach dem Zusammenfügen der beiden Substrate. Nachteil des Wärmehärtungsverfahrens ist, daß sich der Flüssigkristall infolge der Erhitzung ausdehnt und sich seine Viskosität erniedrigt, was eine Durchmischung mit dem Dichtmittel begünstigt.

Beim Lichthärtungsverfahren werden zwei Arten von Dichtmittel unterschieden und zwar solche vom kationischen Polymerisationstyp und solche vom radikalischen Polymerisationstyp, jeweils nach der Art des Photopolymerisationsinitiators. Bei Dichtmitteln vom kationischen Polymerisationstyp besteht das Problem, daß während der Lichthärtung Ionen erzeugt werden, die in den Flüssigkristall diffundieren können, was zu einer Abnahme des spezifischen Widerstands des Flüssigkristalls führt. Bei Dichtmitteln vom radikalischen Polymerisationstyp besteht das Problem, daß die Aushärtungsschrumpfung relativ groß ist, was zu einer geringen Stärke der Adhäsion führen kann. Beiden Arten von Dichtmitteln, d.h. sowohl des kationischen Polymerisationstyps und des radikalischen Polymerisationstyps, ist das Problem gemeinsam, daß schattiert Teile, die nicht mit Licht bestrahlt werden, ungehärtet oder nur unvollständig gehärtet bleiben, beispielsweise hinter Metallkontakten elektronischer Bauelemente des TFT-Arraysubstrats der Flüssigkristallanzeige oder hinter der Schwarzmatrix des Farbfiltersubstrats.

Daher hat sich in der Praxis das Licht-und-Wärmehärtungsverfahren als besonders geeignet erwiesen. Dieses ist dadurch gekennzeichnet, daß das Dichtmittel für Flüssigkristalle zwischen den Substraten einer primären Härtung durch Bestrahlen mit Licht unterzogen wird, gefolgt von einem sekundären Härtungsschritt durch Erwärmen. Dabei ist essentiell, daß der Flüssigkristall beim Licht-und-Wärmehärtungsverfahren sowohl vor und nach dem Belichten als auch vor und nach dem Erwärmen nicht verunreinigt wird. Insbesondere erforderlich sind Maßnahmen zur Aushärtung der oben erwähnten abschattierten Bereiche sowie Maßnahmen zur Verhinderung der Diffusion von Bestandteilen des Dichtmittels während der Wärmehärtung.

Geeignete Lösungsansätze sind eine schnelle Härtung bei niedriger Temperatur vor Beginn einer Durchmischung des Dichtmittels mit dem Flüssigkristall oder die Verwendung von Komponenten im Dichtmittel, die sich weniger oder nicht im Flüssigkristall lösen. Eine schnelle Härtung bei niedriger Temperatur bedeutet, daß die Haltbarkeit des Dichtmittels aufgrund der hohen Reaktivität sehr kurz ist, was in der Praxis ein großes Problem darstellt. Deshalb werden bevorzugt Dichtmittel eingesetzt, die eine lange Haltbarkeit aufweisen und Komponenten enthalten, die im Flüssigkristall schwer löslich sind.

Dichtmittel für Flüssigkristallanzeigen sind beispielsweise in US2007/096056 A1, EP 1780587 A1, US 2003/0147034 A1 und EP 2381304 A1 offengelegt.

Um die Löslichkeit der Dichtmittel im stark unpolaren Flüssigkristall zu verringern, wurden Dichtmittel auf der Basis von polaren Epoxy-Harzen und polaren Acryl-Harzen vorgeschlagen, die alkoholische Hydroxylgruppen oder Sulfongruppen enthalten, wie beispielsweise in der WO 2004/104683, EP 1559735 A1 und US 2008/0305707 beschrieben.

Die oben beschriebenen Probleme der Schädigung des Flüssigkristalls während des Härtens von Dichtmitteln für Flüssigkristallanzeigen sind insbesondere gravierend, wenn der Flüssigkristall selbst polymerisierbare Komponenten enthält: Dabei handelt es sich um einen relativ neuen Display-Modus, den sog. PS ("polymer sustained") oder PSA ("polymer sustained alignment") Modus, der gelegentlich auch Polymer-stabilisiert genannt wird. In PSA-Anzeigen wird ein Flüssigkristallmedium verwendet, das aus einem Flüssigkristall-Host und einer geringen Menge - typischerweise < 1Gew.% - einer oder mehrerer polymerisierbarer Verbindungen besteht. Im Anschluß an das Befüllen des Displays werden die polymerisierbaren Verbindungen in situ polymerisiert und quervernetzt, üblicherweise durch UV-Bestrahlung und unter Anlegen einer Spannung. Besonders geeignet haben sich polymerisierbare Flüssigkristalle als reaktive Komponenten erwiesen, sogenannte reaktive Mesogene (RM).

Der PSA-Modus findet mittlerweile in verschiedenen herkömmlichen LCD-Typen Anwendung wie zum Beispiel PS-VA ("vertically aligned"), PS-OCB-("optically compensated bend"), PS-IPS ("In-Plane-Switching"), PS-FFS ("fringe field switching"), und PS-TN-("twisted nematic"). Die Polymerisation der RM erfolgt im Falle von PS-VA- und PS-OCB-Displays vorzugsweise unter Anlegung einer Spannung, und mit oder ohne, vorzugsweise ohne angelegte Spannung bei PS-IPS-Displays, wodurch ein Pretilt-Winkel der LC-Moleküle in der Anzeigezelle erzeugt wird. Bei PS-OCB-Anzeigen zum Beispiel ist es möglich, die Krümmungsstruktur zu stabilisieren, so daß eine Off-Set-Spannung unnötig ist oder verringert werden kann. Bei PS-VA-Anzeigen hat der Pretilt eine positive Wirkung auf die Schaltzeiten. Diese Beispiele machen deutlich, daß die Polymerstabilisierung einen wesentlichen Einfluß auf die empfindlichen Prozesse beim Schalten nimmt und daß die Polymerisation unter extrem kontrollierten Bedingungen verlaufen muß. Daher ist besonders bei PSA-Anzeigen eine Kontamination des Flüssigkristalls mit Komponenten des Dichtmittels bei der Herstellung kritisch, weil dies zu unkontrollierten Reaktionen der RM und folglich wegen lokaler Fehlorientierung der Flüssigkristallmoleküle zu deutlich sichtbaren Displayfehlern führt.

PS-VA-Anzeigen sind beispielsweise in EP1170626 A2, US6861107, US7169449, US2004/0191428A1, US2006/0066793A1 und US2006/0103804A1 beschrieben. PS-OCB-Anzeigen sind beispielsweise in T.-J- Chen et al., Jpn. J. Appl. Phys. 45, 2006, 2702-2704 and S. H. Kim, L.-C- Chien, Jpn. J. Appl. Phys. 43, 2004, 7643-7647 beschrieben. PS-IPS-Anzeigen sind beispielsweise in US 6177972 und Appl. Phys. Lett. 1999, 75(21), 3264 beschrieben. PS-TN-Anzeigen sind beispielsweise in Optics Express 2004, 12(7), 1221 beschrieben.

Die in Dichtmitteln für Flüssigkristalle eingesetzten Photoinitiatoren sind deswegen besonders problematisch, weil sie nach Aktivierung die eigentlichen reaktiven, die Polymerisation auslösenden oder beschleunigenden Radikale freisetzen, die als kleine Moleküle über eine hohe Mobilität verfügen und üblicherweise in Flüssigkristallen gut löslich sind.

In der US 2009/0147206 werden polymere Photoinitiatoren vorgeschlagen, die aufgrund ihrer auf einem Polyacrylat basierenden Polymerstruktur während des Härtens im Dichtmittel verbleiben sollen. Ein Nachteil ist hier, daß große polymere Photoinitiatoren eine geringe Mobilität aufweisen, und aufgrund der Tatsache, daß sie durch Polymerisation einer Monomervorstufe hergestellt werden, sind besonders reaktive Initiatortypen nur schwer zugänglich.

Aufgabe der vorliegenden Erfindung ist es daher, Photoinitiatoren für die Verwendung in Dichtmitteln für Flüssigkristallanzeigen bereitzustellen, die die oben erwähnten Nachteile nicht oder in nur geringem Ausmaß zeigen.

Überraschend wurde gefunden, daß diese Aufgabe gelöst werden kann, indem Photoinitiatoren der Formel I verwendet werden.

Gegenstand der Erfindung ist daher die Verwendung von Verbindungen der Formel I als Photopolymerisationsinitiatoren von polymerisierbaren Stoffgemischen, die ungesättigte Verbindungen enthalten, oder zur photochemischen Vernetzung von linearen Polymeren, in Dichtmitteln für Flüssigkristallanzeigen, 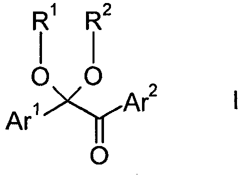 worin
- R¹, R²: unabhängig voneinander ein geradkettiger oder verzweigter oder cyclischer Alkyl-, Cycloalkylalkyl-, Arylalkyl, Alkenyl- oder Cycloalkylalkenylrest mit bis zu 20 C-Atomen oder ein Arylalkenylrest mit 8 bis 20 C-Atomen, in denen jeweils ein oder mehrere CH₂-Gruppen durch -CO- , -O- und/oder -S-so ersetzt sein können, daß keine O- oder S-Atome benachbart sind und in denen ein oder mehrere Wasserstoffatome durch Halogen ersetzt sein können, oder beide Reste R¹ und R² gemeinsam ein zweibindiges Brückenglied W,
- W: ein zweiwertiger Rest eines aliphatischen, geradkettigen oder verzweigten Diols mit 2 bis 20 C-Atomen,
- Ar¹, Ar²: unabhängig voneinander jeweils durch einen oder mehrere Substituenten L substituierte Arylreste, die zusätzlich durch Halogen, Alkyl, Alkoxy mit jeweils 1 bis 5 C-Atomen, oder Phenyl substituiert sein können,
- L: bei jedem Auftreten gleich oder verschieden ein hydrophiler Rest
bedeuten.

Weiterer Gegenstand der Erfindung sind Dichtmittel, die eine oder mehrere Verbindungen ausgewählt aus der Gruppe der Verbindungen der Formel I enthalten.

Die nachfolgend aufgeführte Verbindung I-1-1a ist als Zwischenstufe zur Synthese oberflächenaktiver Photoinitiatoren in WO 02/48203 A1 offengelegt.

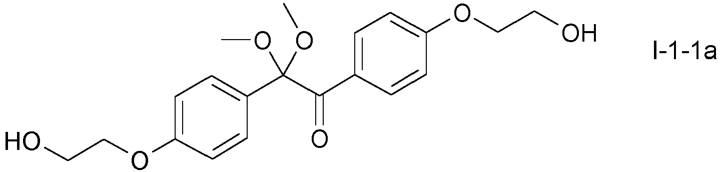

Dichtmittel im Sinne der vorliegenden Erfindung ist ein in flüssigem Zustand verarbeitbares Material, das thermisch, photochemisch, oder thermisch und photochemisch aushärtbar ist und nach dem Aushärten das Austreten von Flüssigkeiten aus einer Öffnung verhindert.

Photopolymerisationsinitiator, order kurz: Photoinitiator im Sinne der vorliegenden Erfindung ist eine Substanz, die unter der Wirkung aktinischer Strahlung eine Polymerisation einzuleiten und zu beschleunigen vermag.

Unter aktinischer Strahlung ist hier und im Folgenden elektromagnetische Strahlung wie nahes Infrarot, sichtbares Licht, UV-Strahlung oder Röntgenstrahlung, insbesondere UV-Strahlung, und Korpuskularstrahlung wie Elektronenstrahlung zu verstehen.

Harz im Sinne der vorliegenden Erfindung ist eine weiche oder hochviskose Substanz oder Substanzgemisch bestehend aus polymerisierbaren Monomeren, Oligomeren oder quervernetzbaren Polymeren, das durch Polymerisation härtbar ist.

Ein radikalisch härtbares Harz im Sinne der vorliegenden Erfindung ist ein photopolymerisierbares Harz, das radikalisch polymerisierbare funktionelle Gruppen enthält, wie beispielsweise (Meth)acrylat- oder Allylgruppen.

In der vorliegenden Anmeldung umfassen sämtliche Atome auch ihre Isotope. Insbesondere können ein oder mehrere Wasserstoffatome (H) durch Deuterium (D) ersetzt sein, was in einigen Ausführungsformen besonders bevorzugt ist; ein hoher Deuterierungsgrad ermöglicht oder erleichtert den analytischen Nachweis von Verbindungen, insbesondere im Falle geringer Konzentrationen.

Halogen bedeutet F, Cl, Br oder I, bevorzugt F oder Cl.

Falls R¹ oder R², nachfolgend allgemein R genannt, einen Alkylrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, n-Propyl, n-Butyl, n-Pentyl, n-Hexyl, n-Heptyl, ferner Methyl, n-Octyl, n-Nonyl, n-Decyl, n-Undecyl, n-Dodecyl, n-Tridecyl, n-Tetradecyl, n-Pentadecyl.

Falls R einen Alkylrest bedeutet, in dem eine CH₂-Gruppe durch O ersetzt ist, bedeutet dieser vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkenylrest bedeutet, so kann dieser geradkettig oder verzweigt sein. Vorzugsweise ist er geradkettig und hat 2 bis 10 C-Atome.

Er bedeutet demnach besonders Vinyl, Prop-1-, oder Prop-2-enyl, n-But-1-, 2- oder n-But-3-enyl, n-Pent-1-, 2-, 3- oder n-Pent-4-enyl, n-Hex-1-, 2-, 3-, 4- oder n-Hex-5-enyl, n-Hept-1-, 2-, 3-, 4-, 5- oder n-Hept-6-enyl, n-Oct-1-, 2-, 3-, 4-, 5-, 6- oder n-Oct-7-enyl, n-Non-1-, 2-, 3-, 4-, 5-, 6-, 7- oder n-Non-8-enyl, n-Dec-1-, 2-, 3-, 4-, 5-, 6-, 7-, 8- oder n-Dec-9-enyl.

Falls R einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyl-oxypropyl, 4-Acetyl-oxybutyl, Methoxycarbonyl, Ethoxycarbonyl, Propoxy-carbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxy-carbonyl)-propyl oder 4-(Methoxycarbonyl)-butyl.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verzweigte Reste R enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Der Begriff Aryl im Sinne der vorliegenden Erfindung bedeutet aromatische Kohlenwasserstoffreste oder deren Derivate. Diese können monocylisch, polycyclisch oder kondensiert sein, d.h. für sich (z.B. Phenyl), durch andere Arylreste substituiert (z. B. Biphenyl) oder anelliert (z.B. Naphthalin) sein. Desgleichen können sie eine Kombination dieser Merkmale aufweisen (z.B. Phenylnaphthalin).

Bevorzugte Arylreste bestehen aus einem , zwei oder drei Ringelementen mit 6 bis 25 C-Atomen, die optional substituiert und anellierte aliphatische Ringe enthalten können.

Bevorzugte Arylreste sind beispielsweise abgeleitet aus den Verbindungen Benzol, Biphenyl, Terphenyl, Naphthalin, Tetrahydronaphthalin, Anthracene, Binaphthyl, Phenanthren, 9,10-Dihydro-phenanthren, Pyren, Dihydropyren, Fluoren, Indan, Indenofluoren, Spirobifluoren, etc.

Die oben und unten erwähnten Arylreste können weiterhin durch Alkyl, Alkoxy, Thioalkyl, Fluor, Fluoroalkyl oder weitere Arylgruppen substituiert sein.

Die Cycloalkylreste umfassen sowohl gesättigte als auch ungesättigte ringe, d.h. solche, die ausschließlich Einfachbindungen enthalten und solche die eine oder mehrere Doppel- oder Dreifachbindungen enthalten, ohne dabei aromatisch zu sein. Heterocyclen enthalten ein oder mehrere Heteroatome, bevorzugt ausgewählt aus S, N, O, Se, Si.

Die (nicht aromatischen) alicylischen oder heterocyclischen Reste können monocyclisch sein, enthalten also einen Ring (z.B. Cyclohexan) oder Polycyclisch, d.h. sie enthalten mehrere Ringe, wie z.B. Decalin oder Bicyclooctan. Besonders bevorzugt sind gesättigte Ringe. Weiterhin bevorzugt sind mono-, bi- oder tricyclische Gruppen mit 3 bis 25 Ringatomen, die optional anelliert und optional substituiert sein können. Weiterhin bevorzugt sind 5-, 6-, 7- oder 8-gliedrige cyclische Kohlenwasserstoffe, in denen ein oder mehrere CH₂-Gruppen durch Si und/oder ein oder mehrere CH- oder CH₂-Gruppen durch N und/oder ein oder mehrere nicht benachbarte CH₂-Gruppen durch O oder S ersetzt sind. Bevorzugte Substituenten der alicyclischen Reste sind Alkyl oder Alkenylreste wie oben definiert.

Bevorzugte alicyclische und heterocyclische Gruppen sind beispielsweise abgeleitet von den Grundkörpern Cyclopentan, Tetrahydrofuran, Tetrahydrothiophen, Pyrrolidin, Cyclohexan, Silinan, Cyclohexen, Tetrahydropyran, Tetrahydrothiopyran, 1,3-Dioxan, 1,3-Dithiane, Piperidin, Cycloheptan, und kondensierte Ringe wie Tetrahydronaphthalin, Decalin, Indan, Bicyclo[1.1.1]pentan, Bicyclo[2.2.2]octan, Spiro[3.3]heptan, Octahydro-4,7-methanoindan.

Der erfindungsgemäße Photoinitiator kann entweder allein oder in Kombination mit zwei oder mehr Verbindungen davon verwendet werden. Der erfindungsgemäße Photoinitiator kann zusammen mit einem oder mehreren Sensibilisatoren und einem oder mehreren weiteren Photoinitiatoren verwendet werden. Die Wahl, Kombination und das Mischverhältnis des Photopolymerisationsinitiators und des Sensibilisators kann in geeigneter Weise gemäß dem ultraviolett-aushärtenden Monomer und des zu verwendenden Geräts bestimmt werden.

Typische Beispiele für weitere Photopolymerisationsinitiatoren und Sensibilisatoren umfassen Acetophenon, 2,2-Diethoxyacetophenon, p-Dimethylaminoacetophenon, p-Dimethylaminopropiophenon, Benzophenon, 2-Chlorbenzophenon, p,p'-Dichlorbenzophenon, p,p'-Bisdiethylaminobenzophenon, Michlers Keton, Benzil, Benzoin, Benzoinmethylether, Benzoinethylether, Benzoinisopropylether, Benzoin-n-propylether, Benzyldimethylketal, Tetramethylthiurammonosulfid, Thioxanthon, 2-Chlorthioxanthon, 2-Methylthioxanthon, Azobisisobutyronitril, Benzoinperoxid, Di-tert-butylperoxid, 1-Hydoxycyclohexylphenylketon, 2-Hydroxy-2-methyl-1-phenylpropan-1-on, 1-(4-Isopropylphenyl)-2-hydroxy-2-methylpropan-1-on und Methylbenzoylformat, sowie die kommerziell erhältlichen Produkte Irgacure651®, Irgacure184®, Irgacure907®, Irgacure369® oder Darocurel 173® (Ciba AG).

Die verwendete Menge liegt im allgemeinen bei 0,1 bis 10 Gew-%, bezogen auf das Gesamtgewicht des ultraviolett-härtenden Harzes. Als Photopolymerisationsinitiatoren vom Typ kationische Polymerisation können Initiatoren wie aromatische Diazoniumsalze, aromatische Haloniumsalze, aromatische Sulfoniumsalze und Metallocenverbindungen verwendet werden. Spezifische Beispiele dafür umfassen Triphenylsulfoniumhexafluorophosphat, Diphenyliodiniumhexafluorantimonat und dergleichen. Das Monomer vom Typ kationische Polymerisation wird vorzugsweise innerhalb eines Bereiches von 80°C bis 170°C, insbesondere von 100°C bis 150°C für die vollständige Aushärtung erhitzt. Die Dauer des Erhitzens liegt in der Regel zwischen 5 und 30 Minuten, was nach den Bedingungen variieren kann.

Ein hydrophiler Rest im Sinne der vorliegenden Erfindung ist ein durch eine oder mehrere Hydroxylgruppen und/oder Epoxygruppen substituierter geradkettiger oder verzweigter oder cyclischer Alkylrest, in dem eine oder mehrere CH₂-Gruppen durch -O- oder -NR⁰- so ersetzt sein können, daß keine O-Atome benachbart sind, in dem eine oder mehrere CH₂-Gruppen durch -NH- ersetzt sein können und in dem in Verzweigungen eine oder mehrere >CH--Gruppen durch >N- ersetzt sein können.

Beispiele für typische hydrophile Reste sind die nachfolgend für L angegebenen Gruppen.

In einer bevorzugten Ausführungsform werden Photoinitiatoren der Formel I verwendet, in denen
- R¹, R²: unabhängig voneinander ein geradkettiger oder verzweigter Alkylrest mit 1 bis 7 C-Atomen,
- W: -(CH₂)₂-, -(C(CH₃)₂)₂-, -(CH₂)₃-, -CH₂C(CH₃)₂CH₂-,
- Ar¹, Ar²: unabhängig voneinander jeweils durch einen Substituenten L substituiertes 1,4-Phenylen,
- L: -CH₂-L', -O-L' oder -N(L')₂
- L': ein geradkettiger oder verzweigter Alkylrest mit 1 bis 20 C-Atomen, in dem eine oder mehrere CH₂-Gruppen durch Cycloalkandiylreste mit 3 bis 8 Ringatomen ersetzt sein können und in dem eine oder mehrere nicht benachbarte CH₂-Gruppen durch O ersetzt sein können und in dem ein oder mehrere H-Atome durch -OH oder 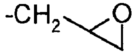 ersetzt sind.
bedeuten.

L bedeutet bevorzugt -(CH₂)ₚOG, -O(CH₂)ₘ₊₁OG, -(CH₂)ₙ(OCH₂CH₂)ₘOG, -(O)ₙ(CH₂)ₘCH(OH)CH₂OG, -O(CH₂CH₂)ₘ₊₁OCH₂CH(OG)CH₂OG, -OC(CH₂OG)₃, -OC(CH₂OG)₂(CH₂)ₙH, -(CH₂)ₙOCH₂CH(OH)CH₂OG, -(OCH₂CH₂)ₘOCH₂CH(OG)CH₂OG, -(CH₂)ₙOCH₂(CH)ₘ((CH₂)ₘOG)₂, -(CH₂)ₙOC(CH₂OG)₃, --(CH₂)ₘOC(CH₂OG)₂(CH₂)ₙH, -N((CH₂)ₘ₊₁OG)₂, -N((CH₂CH₂O)ₘG)₂, -N((CH₂)ₘCH(OG)CH₂OG)₂ und 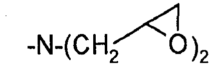 worin
- m: eine ganze Zahl von 1 bis 10,
- n, p: jeweils unabhängig voneinander eine ganze Zahl von 0 bis 10
- G: H, 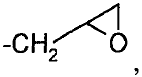 oder ein Monosaccharidrest ausgewählt aus Glycopyranose und Glycofuranose, vorzugsweise Glucose;
besonders bevorzugt H, mit der Maßgabe, daß wenn p gleich 0 ist, G nicht H sein kann,
bedeuten.

Besonders bevorzugt sind Verbindungen der Formeln

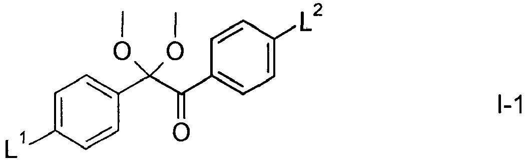

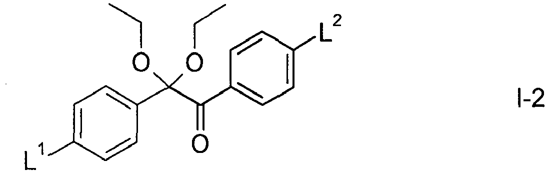

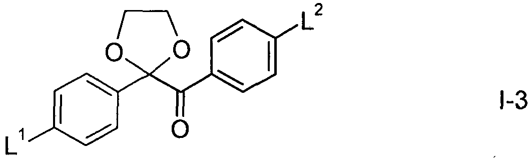

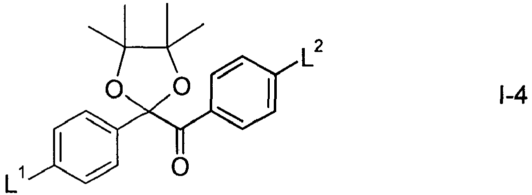

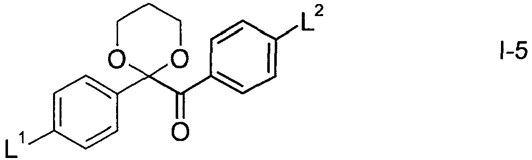

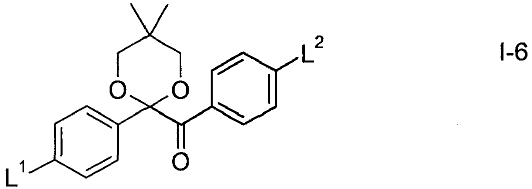

In einer weiteren bevorzugten Ausführungsform sind die Verbindungen der Formel I ausgewählt aus den Verbindungen der Formel I-1. Besonders bevorzugte Unterformeln der Formel I-1 sind die Unterformeln I-1-1 bis I-1-14. 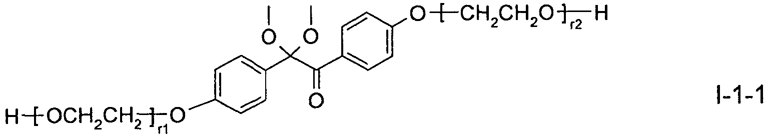 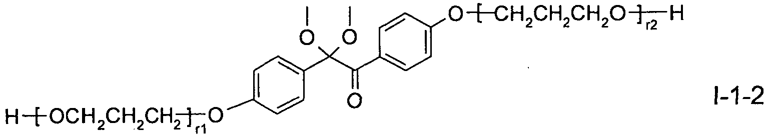 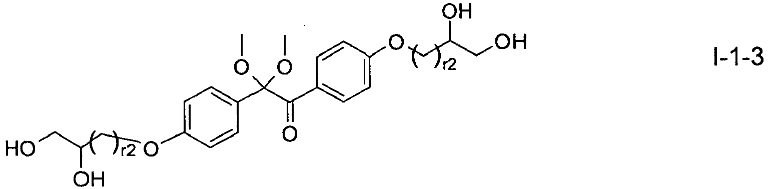 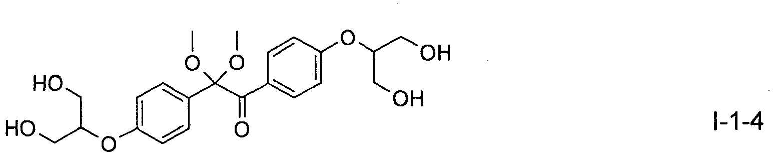 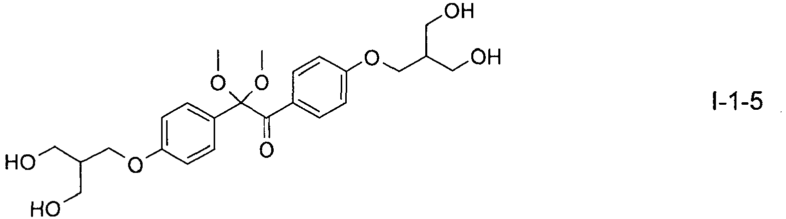 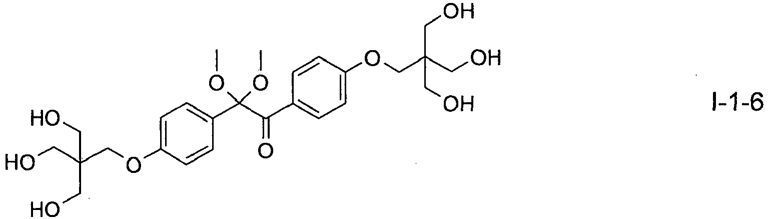 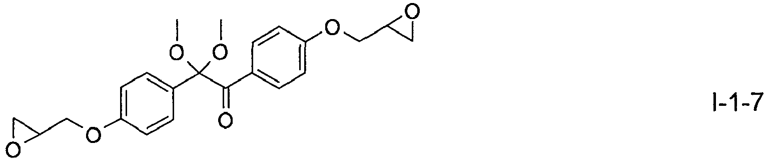 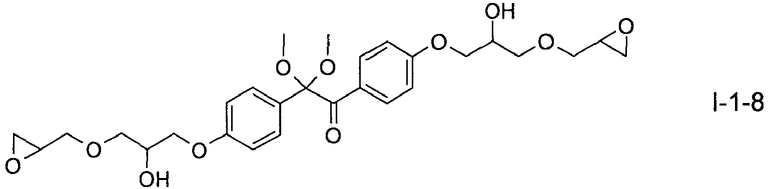 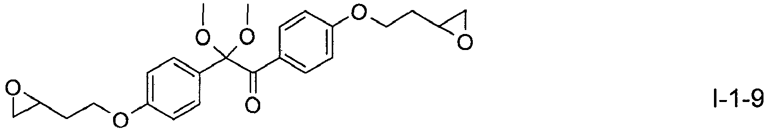 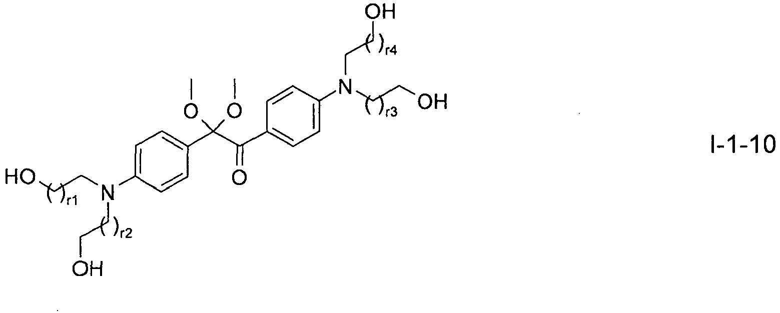 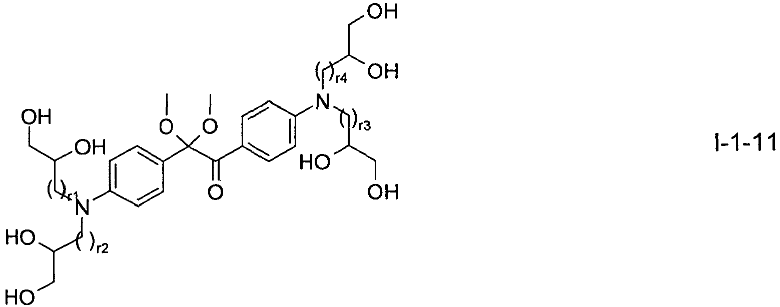 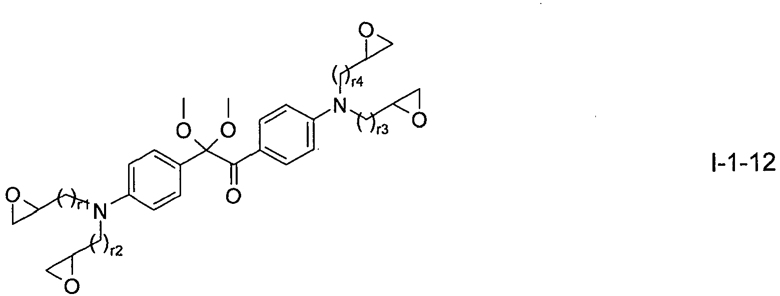 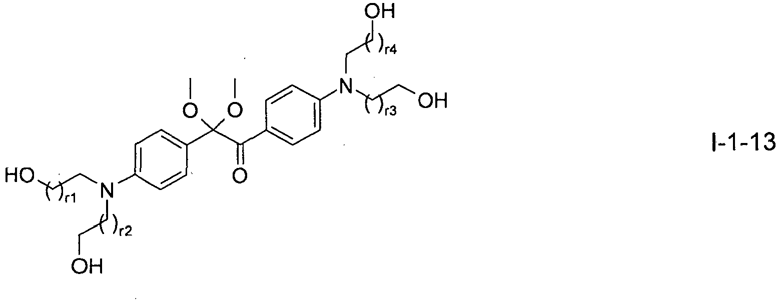 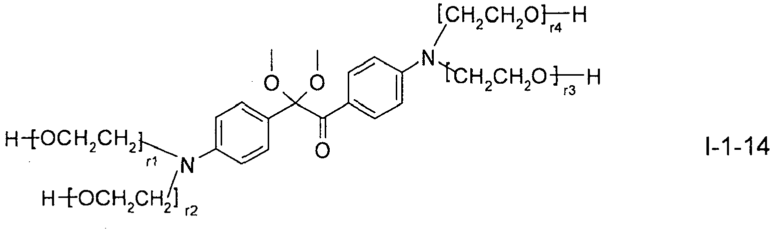 worin
r1, r2, r3 und r4 unabhängig voneinander 1, 2 oder 3, bevorzugt 1
bedeuten.

In einer bevorzugten Ausführungsform der vorliegenden Erfindung werden solche Verbindungen der Formel I verwendet, deren nach A. K. Ghose, A. Pritchett und G. M. Crippen, J. Comput. Chem. 1988, 9, 80-90 berechneter Octan-1-ol-Wasser-Verteilungskoeffizienten einen Wert von log P ≤ 3.2 aufweist.

Bevorzugt ist log P größer 0,1 und kleiner 3,2, besonders bevorzugt größer 0,25 und kleiner 3,0, ganz besonders bevorzugt größer 0,5 und kleiner 2,8.

In der nachfolgenden Tabelle 1 sind Beispiele für besonders bevorzugte Verbindungen der Formel I und deren berechnete logP-Werte aufgeführt.

**Tabelle 1. Beispiele und deren logP-Wert**

| Nr. | Struktur | logP |
|---|---|---|
| 1 | 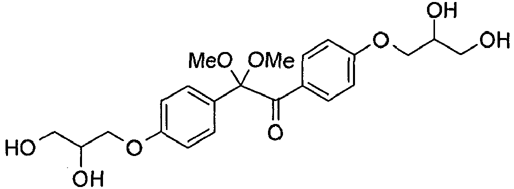 | 1,30 |
| 2 | 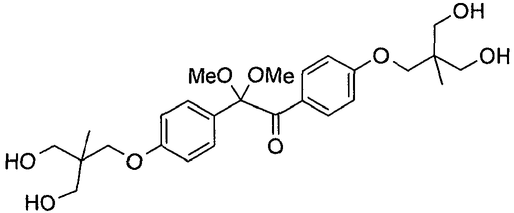 | 2,68 |
| 3 | 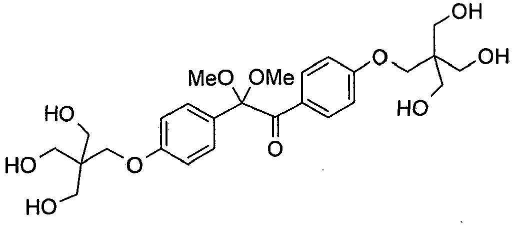 | 0,56 |
| 4 | 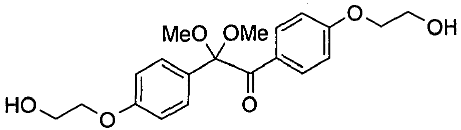 | 2,37 |
| 5 | 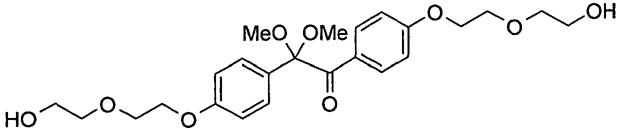 | 2,06 |
| 6 | 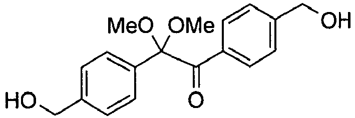 | 2,51 |
| 7 | 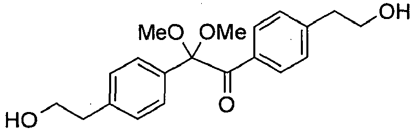 | 3,07 |
| 8 | 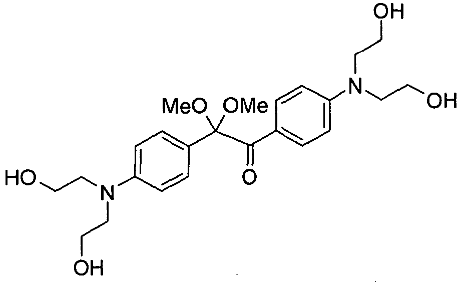 | 2,16 |
| 9 | 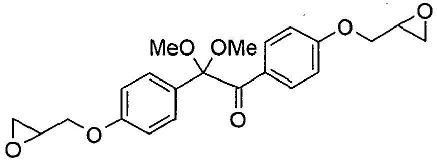 | 2,56 |
| 10 | 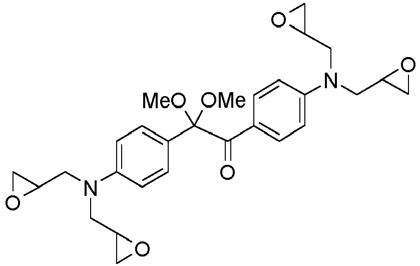 | 2,55 |

Weiterer Gegenstand der Erfindung sind Dichtmittel für Flüssigkristallanzeigen, die eine oder mehrere Verbindungen der Formel I enthalten.

Dichtmittel für Flüssigkristallanzeigen sind aus dem Stand der Technik bekannt und beispielsweise in EP 1 559 735 A1, EP 2 381 304 A1, EP 1 780587A1 und EP 2 586 827 A1 offengelegt. Vorzugsweise sind die erfindungsgemäßen Dichtmittel photochemisch aushärtbar. Besonders bevorzugt sind die erfindungsgemäßen Dichtmittel sowohl photochemisch als auch thermisch aushärtbar.

Die erfindungsgemäßen Dichtmittel enthalten bevorzugt a) eine oder mehrere härtbare Harze, die Verbindungen enthalten, die jeweils durch eine oder mehrere Epoxy-gruppen substituiert sind (nachfolgend "Epoxide" genannt); und/oder b) eine oder mehrere Verbindungen, die jeweils durch eine oder mehrere Acrylsäure- oder Methacrylsäureestergruppen substituiert sind (nachfolgend "(Meth)acrylate" genannt), und/oder c) eine oder mehrere Verbindungen, die sowohl durch sowohl Epoxy- als auch durch (Meth)acrylsäureestergruppen substituierte sind (nachfolgend "Epoxyacrylate" genannt).

Die einzelnen Komponenten a), b) und c) können dabei sowohl als Monomere als auch als Oligomere vorliegen. Die Wahl der Komponenten ist grundsätzlich nicht auf bestimmte Verbindungen beschränkt. Bevorzugt sind die polymerisierbaren Verbindungen di-, tri oder polyreaktiv, d.h. sie enthalten zwei, drei oder mehr reaktive Epoxid- und/oder (Meth)acrylatgruppen. Im Hinblick auf eine möglichst geringe Kontamination des Flüssigkristalls mit den Komponenten des Dichtmittels sind Harze bevorzugt, die Hydroxylgruppen, Sulfongruppen oder Ethergruppen enthalten.

Bevorzugt enthalten die erfindungsgemäßen Dichtmittel neben einer oder mehreren Verbindungen der Formel I
i) ein radikalisch härtbares Harz
ii) ein Epoxidharz
iii) einen Epoxid-Härter

Beispiele für radikalisch härtbare Harze sind (Meth)acrylate und ungesättigte Polyesterharze, die jedes für sich oder im Gemisch von einem oder mehreren solcher Substanzen verwendet werden können. Vorzugsweise besitzen die monomeren (Meth)acrylate zwei oder mehrere (Meth)acrylatgruppen pro Molekül.

Beispiele für (Meth)acrylate sind Urethan-(Meth)acrylate enthaltend eine Urethan-Brücke sowie Epoxy-(Meth)acrylate, die neben einer oder mehreren (Meth)acrylat-Gruppen eine oder mehrere Glycidyl-Gruppen tragen.

Beispiele für Urethan-(Meth)acrylate sind Verbindungen, die durch Reaktion zwischen Diisocyanaten (z.B. Isophoron-diisocyanat) mit Verbindungen erhalten werden, die an Isocyanate addieren können, wie z.B. Acrylsäure oder Hydroxyethylacrylat.

Diese Derivate können Kettenverlängerungen durch Caprolacton oder Polyole beinhalten und sind kommerziell beispielsweise unter den Handelsnamen U-122P, U-3, 40P, U-4HA und U-1084A (Shin-Nakamura Chemical), sowie KRM 7595, KRM 7610 und KRM 7619 (Daicel Cytec Co.) erhältlich.

Beispiele für Epoxy-(Meth)acrylate sind Derivate von Epoxidharzen (z.B. Bisphenol-A-Epoxidharze oder Propylenglycoldiglycidylether) und (Meth)acrylsäure und sind beispielsweise kommerziell unter den Handelsnamen EA-1020, EA-6320, und EA-5520 (Shin-Nakamura Chemical), sowie EPOXY ESTER 70PA und EPOXY ESTER 3002A (Kyoiesha Chemical) erhältlich. Weitere Beispiele für geeignete (Meth)acrylate sind Methylmethacrylat, Tetrahydrofurfurylmethacrylat, Benzylmethacrylat, Isobornylmethacrylat, 2-Hydroxyethylmethacrylat, Glycidylmethacrylat, (Poly)ethylenglycoldimethacrylat, 1,4-Butandioldimethacrylat, 1,6-Hexandioldimethacrylat, Trimethylolpropantriacrylat, Pentaerythritoltriacrylat und Glycerindimethacrylat.

Die Gruppe der radikalisch härtbaren Harze umfaßt ebenso Epoxyacrylate, die eine oder mehrere (Meth)acrylatgruppen und eine oder mehrere Epoxygruppen pro Molekül enthalten und die dadurch hergestellt werden, daß die oben beschriebenen Epoxyharze teilweise mit (Meth)acrylsäure in Gegenwart einer Base umgesetzt werden; weiterhin umfaßt sind Verbindungen, die dadurch hergestellt werden, daß bi- oder höherfunktionale Isocyanate mit einem halben Äquivalent eines Hydroxylgruppen enthaltenden (Meth)acrylats und anschließend mit einem halben Äquivalent Glycidol umgesetzt werden; weiterhin umfaßt sind Verbindungen, die dadurch hergestellt werden, daß ein durch eine Isocyanatgruppe substituiertes Methacrylat mit Glycidol umgesetzt wird.

Solche Materialien sind z.B. unter dem Handelsnamen UVAC1561 (Daicel Cytec) und 4HBAGE (Nippon Kasei) erhältlich.

Beispiele für latente Epoxid-Härter gemäß der vorliegenden Erfindung sind Dicyandiamide, modifizierte Polyamine, Hydrazide, 4,4'-Diaminodiphenylsulfon, Bortrifluorid- Aminkomplexe, sowie Imidazol, Guanidin, Harnstoff, Melamin und deren Derivate. Beispiele für die modifizierten Polyamine sind Epoxy-Addukte von Polyaminen, Amide von Polyaminen und Mannich-modifizierte Polyamine. Beispiele für Polyamine sind aliphatische Polyamine wie z.B. Ethylendiamin, Diethylentriamin, Triethylentetramin, Tetraethylenpentamin, 1,2-Diaminopropan, Polyoxypropylendiamin und Polyoxypropylentriamin; alicyclische Polyamine wie beispielsweise Isophorondiamin, Menthendiamin, Bis(4-amino-3-methyldicyclohexyl)methan, Diaminodicyclohexylmethan, Bis(aminomethyl)cyclohexan, N-aminoethylpiperazin und 3,9-Bis(3-aminopropyl)-2,4,8,10-tetraoxaspiro[5,5]undecan; aromatische Polyamine, wie z. B. m-Phenylendiamin, p-Phenylendiamin, Tolylen-2,4-diamin, Tolylene-2,6-diamin, Mesitylen-2,4-diamin, Mesitylen-2,6-diamin, 3,5-Diethyltolylen-2,4-diamin und 3,5-Diethyltolylene-2,6-diamin, Biphenylendiamin, 4,4-Diaminodiphenylmethan, 2,5-Naphthylendiamin und 2,6-Naphthylendiamin; und Imidazole, wie beispielsweise 2-Methylimidazol, 2-Ethyl-4-methylimidazol, 2-Isopropylimidazol, 2-Undecylimidazol, 2-Heptadecylimidazol, 2-Phenylimidazol, 2-Phenyl-4-methylimidazol und 2-Aminopropylimidazol.

Die Epoxyaddukte werden durch dem Fachmann geläufige Addition von Epoxiden an Polyamine hergestellt. Die Epoxide sind vorzugsweise aliphatische Verbindungen, aromatische Verbindungen, etc. Diese Verbindungen können jeweils für sich oder im Gemisch mit anderen Epoxiden eingesetzt werden.

Beispiele für alicyclische Epoxide sind Polyglycidylether von Polyolen enthaltend mindestens einen Alicyclus, Cyclohexenoxid- oder Cyclopentenoxid und deren Derivate, insbesondere gesättigte Derivate von Bisphenol-A-diglycidylether, 3,4-Epoxycyclohexylmethyl-3,4-epoxycyclohexancarboxylat, 3,4-Epoxy-1-methylcyclohexyl-3,4-epoxy-1-methylcyclohexancarboxylat, 6-Methyl-3,4-epoxycyclohexylmethyl-6-methyl-3,4-epoxycyclohexancarboxylat, 3,4-Epoxy-3-methylcyclohexylmethyl-3,4-epoxy-3-methylcyclohexancarboxylate, 3,4-Epoxy-5-methylcylcohexylmethyl-3,4-epoxy-5-methylcyclohexancarboxylat, 2-(3,4-Epoxycyclohexyl-5,5-spiro-3,4-epoxy)cyclohexan-metadioxan, Bis(3,4-epoxycyclohexylmethyl)adipat, 3,4-Epoxy-6-methylcyclohexyl-carboxylat, Methylenbis(3,4-epoxycyclohexane), Dicyclopentadiendiepoxid, Ethylenbis(3,4-epoxycyclohexancarboxylat), Dioctylepoxyhexahydrophthalat und Di-2-ethylhexyl-epoxyhexahydrophthalat.

Kommerziell erhältliche Produkte, die solche Verbindungen enthalten, sind beispielsweise UVR-6100, UVR-6105, UVR-6110, UVR-6128 und UVR-6200 (Union Carbide); Celloxide 2021, Celloxide 2021P, Celloxide 2081, Celloxide 2083, Celloxide 2085, Celloxide 2000, Celloxide 3000, Cyclomer A200, Cyclomer M100, Cyclomer M101, Epolead GT-301, Epolead GT-302, Epolead 401, Epolead 403, ETHB und Epolead HD300 (Daicel Chemical Industries, Ltd.) und KRM-2110 und KRM-2199 (ADEKA Corp.).

Beispiele für aromatische Epoxide sind Polyglycidylether mehrwertiger Phenole oder deren Alkylenoxid-Addukte, die mindestens einen aromatischen Ring enthalten, wie beispielsweise Glycidylether von Bisphenol-A, Bisphenol-F, oder deren Alkylenoxidaddukte, sowie Epoxy-Novolak-Harze.

Beispiele für aliphatische Epoxide sind Polyglycidylether aliphatischer Polyole oder deren Alkylenoxidaddukte, Polyglycidylester von aliphatischen langkettigen Polycarbonsäuren, Poly(meth)acrylsäureglycidylester, insbesondere 1,4-Butandioldiglycidylether, 1,6-Hexandioldiglycidylether, Glycerintriglycidylether, Trimethylolpropantriglycidylether, Sorbitoltetraglycidylether, Dipentaerythritolhexaglycidylether, Polyethylenglycoldiglycidylether und Polypropylenglycoldiglycidylether; Polyglycidylether von Polyetherpolyolen, hergestellt durch Addition einer oder mehrerer Alkylenoxide an aliphatische Polyole, wie beispielsweise Propylenglycol, Trimethylolpropan und Glycerin; und Diglycidylester von langkettigen aliphatischen Dicarbonsäuren. Weiterhin sind bevorzugt Monoglycidylether von höheren aliphatische Alkoholen, Monoglycidylether von Phenol, Cresol, Butylphenol oder Polyetheralkoholen hergestellt durch Addition von Alkylenoxiden an diese Phenole, Glycidylester höherer Fettsäuren, epoxidiertes Sojabohnenöl, Octylepoxystearat, Butylepoxystearat und epoxidiertes Polybutadien.

Beispiele für kommerziell erhältliche aromatische oder aliphatische Epoxide sind Epikote 801 und Epikote 828 (Yuka Shell Epoxy Co., Ltd.); PY-306, 0163, und DY-022 (Ciba Specialty Chemicals); KRM-2720, EP-3300, EP-4000, EP-4901, EP-4010, EP-4080, EP-4900, ED-505, und ED-506 (ADEKA); Epolite M-1230, Epolite EHDG-L, Epolite 40E, Epolite 100E, Epolite 200E, Epolite 400E, Epolite 70P, Epolite 200P, Epolite 400P, Epolite 1500NP, Epolite 1600, Epolite 80MF, Epolite 100MF, Epolite 4000, Epolite 3002, und Epolite FR-1500 (Kyoeisha Chemical); Santoto ST0000, YD-716, YH-300, PG-202, PG-207, YD-172, und YDPN638 (Tohto Kasei Co., Ltd.); TEPIC-S (Nissan Chemical Industries, Ltd.); und Epichlon N-665, Epichlon N-740, Epichlon HP-7200, und Epichlon HP-4032 (DIC Corp.).

Polyamide werden durch Umsetzung von Polyaminen mit Carbonsäuren wie beispielsweise Adipinsäure, Sebacinsäure, Phthalsäure, Isophthalsäure in der dem Fachmann bekannten Art und Weise erhalten.

Mannich-modifizierte Polyamine werden hergestellt, indem ein Polyamin mit Aldehyden, z.B. Formaldehyd, und Phenolen wie beispielsweise Phenol, Cresol, Xylenol, t-Butylphenol oder Resorcin umgesetzt wird.

Beispiele für Hydrazide sind Oxalsäuredihydrazid, Malonsäuredihydrazid, Bernsteinsäuredihydrazid, Glutarsäuredihydrazid, Adipinsäuredihydrazid, Korksäuredihydrazid, Azelainsäuredihydrazide, Sebacinsäuredihydrazid und Phthalsäuredihydrazid.

Beispiele für Harnstoffderivate sind 3-(3,4-Dichlorophenyl)-1,1-dimethylharnstoff, Isophorondiisocyanat-dimethylharnstoff, und Tolylendiisocyanat-dimethylharnstoff.

Beispiele für Epoxidharze (iii) sindPolyglycidylether mehrwertiger Phenole wie beispielsweise Hydrochinon, Resorcin, Pyrocatechol und Phloroglucinol; Polyglycidylether kondensierter aromatischer Hydroxyverbindungen wie Naphthol, Biphenylol, Methylenenbisphenol (Bisphenol F), Methylenbis(orthocresol), Ethylidenbisphenol, Isopropylidenbisphenol (Bisphenol A), 4,4'-Dihydroxybenzophenon, Isopropylidenbis(orthocresol), Tetrabrombisphenol-A, 1,3-Bis(4-hydroxycumylbenzol), 1,4-bis(4-Hydroxycumylbenzol), 1,1,3-tris(4-Hydroxyphenyl)butan, 1,1,2,2-tetra(4-Hydroxyphenyl)ethan, Thiobisphenol, Sulfobisphenol, Oxybisphenol, Phenol-Novolak, Orthocresol-Novolak, Ethylphenol Novolak, Butylphenol-Novolak, Octylphenol-Novolak, Resorcin-Novolak und Terpendiphenol; weiterhin Polyglycidylether mehrwertiger Alkohole wie Ethylenglycol, Propylenglycol, Butylenglycol, Hexandiol, Polyglycol, Thiodiglycol, Glycerin, Trimethylolpropan, Pentaerythritol, Sorbitol und Bisphenol-A-ethylenoxid-Addukte; Homo-oder Copolymere von Glycidylestern aliphatischer, aromatischer oder alicyclischer mehrbasiger Carbonsäuren wie beispielsweise Maleinsäure, Fumarsäure, Itaconsäure, Bernsteinsäure, Glutarsäure, Korksäure, Adipinsäure, Azelainsäure, Sebacinsäure, Phthalsäure, Isophthalsäure, Terephthalsäure, Trimellitsäure, Pyromellitsäure, Tetrahydrophthalsäure, Hexahydrophthalsäure und endo-Methylentetrahydrophthalsäure, oder Glycidylmethacrylat; weiterhin Epoxide enthaltend eine Glycidylaminogruppe wie z.B. N,N-Glycidylanilin, Bis(4-(N-methyl-N-glycidylamino)phenyl)methan und Diglycidyl-o-toluidin; Epoxidierte cycloalkene wie beispielsweise Vinylcyclohexendiepoxid, Dicyclopentadiendiepoxid, 3,4-Epoxycyclohexylmethyl 3,4-Epoxycyclohexancarboxylat, 3,4-Epoxy-6-methylcyclohexylmethyl-6-methylcyclohexancarboxylat und bis(3,4-Epoxy-6-methylcyclohexylmethyl)adipat; epoxidierte konjugierte Polyene wie z.B. epoxidiertes Polybutadien, epoxidiertes Acrylnitril-Butadien-Copolymer und ein epoxidiertes Styrol-Butadien-Copolymer; weiterhin heterocyclische Verbindungen wie Triglycidylisocyanursäureester.

In einer bevorzugten Ausführungsform enthalten die erfindungsgemäßen Dichtmittel weitere Komponenten, wie beispielsweise anorganische Füllstoffe, oder Silane zur Verbesserung der Klebkraft und der wasserabweisenden Eigenschaften, organische Lösungsmittel, Pigmente, Entschäumer, leitfähige Zusätze, Egalisierungsmittel, etc.

Die Herstellung der erfindungsgemäßen Dichtmittel erfolgt beispielsweise durch Mischen und Lösen von vorbestimmten Mengen der zur Photohärtung eingesetzten Komponenten, der für die thermische Härtung eingesetzten Komponenten, und falls erforderlich, verschiedener Additive und anschließende Homogenisierung der Mischung unter Verwendung einer bekannten Mischvorrichtung, beispielsweise einer Dreiwalzenmühle , einer Sandmühle oder einer Kugelmühle.

Das erfindungsgemäße Dichtmittel ist zum Abdichten von Behältern geeignet, um den Austritt von Flüssigkeiten oder Flüssigkristallen zu verhindern, insbesondere zum Abdichten von Flüssigkristallanzeigen und ganz besonders zum Abdichten von Flüssigkristallanzeigen, die nach dem ODF-Verfahren hergestellt werden. Die Flüssigkeiten oder Flüssigkristalle sind in keiner Weise beschränkt. Das Dichtmittel ist weiterhin geeignet zur Verwendung in organischen Leuchtdioden, organischen Solarzellen, organischen Farbstoffsolarzellen und ähnlichen Bauteilen. Das Dichtmittel ist insbesondere geeignet zum Abdichten von Flüssigkristallanzeigen, die Flüssigkristalle mit polymerisierbaren Komponenten (reaktiven Mesogenen) enthalten und/oder Flüssigkristalleinzelsubstanzen mit ungesättigten Gruppen, wie beispielsweise Alkenylreste oder Cyclohexen-1,4-diyleinheiten. Bevorzugt enthalten die reaktiven Mesogene eine oder mehrere (Meth)acrylatgruppen. Weitere Gegenstand der Erfindung sind Flüssigkristallanzeigen, die unter Verwendung der erfindungsgemäßen Dichtmittel im ODF-Verfahren hergestellt werden.

Zur Herstellung der Flüssigkristallanzeige werden dem Dichtmittel Abstandshalter (engl.: "Spacer") hinzugefügt, um einen genau definierten Abstand der Substrate und somit einen genau definierten Zellspalt der Anzeige herzustellen, je nach Anwendung typischerweise 2-8 µm. Auf eines der beiden Glassubstrate wird zunächst das Dichtmittel aufgetragen, gefolgt von der dem inneren Volumen der fertigen Flüssigkristallanzeige genau entsprechenden Menge an Flüssigkristall. Nach dem Vereinigen der beiden Substrate wird die Anzeige mit einer Dosis von 1000 mJ bis 18000 mJ UV-Licht bestrahlt, wobei die beiden Substrate zusammengepreßt werden. Danach wird das Dichtmittel bei 110 °C bis 140°C für 1 bis 3 h thermisch gehärtet.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, daß man sie aus dem Reaktionsgemisch nicht isoliert, sondern sofort weiter zu den Verbindungen der allgemeinen Formel I umsetzt.

Die Synthesen erfindungsgemäß verwendeter Verbindungen der allgemeinen Formel I werden in den Beispielen exemplarisch beschrieben. Die Ausgangssubstanzen sind nach allgemein zugänglichen Literaturvorschriften oder käuflich zu erhalten.

Besonders geeignete Synthesewege zu den erfindungsgemäß verwendeten Verbindungen werden im Folgenden anhand der Schemata 1-4 erläutert.

Die Synthese des Grundkörpers der erfindungsgemäß verwendeten Verbindungen, d.h. der Monoketale des Benzils, erfolgt beispielsweise nach R. K. Summerbell, D. R. Berger, J. Amer. Chem. Soc. 1959, 81, 633-639 durch Oxidation von Benzilketalen, wie im Schema 1 am Beispiel des Ethylenketals verdeutlicht.

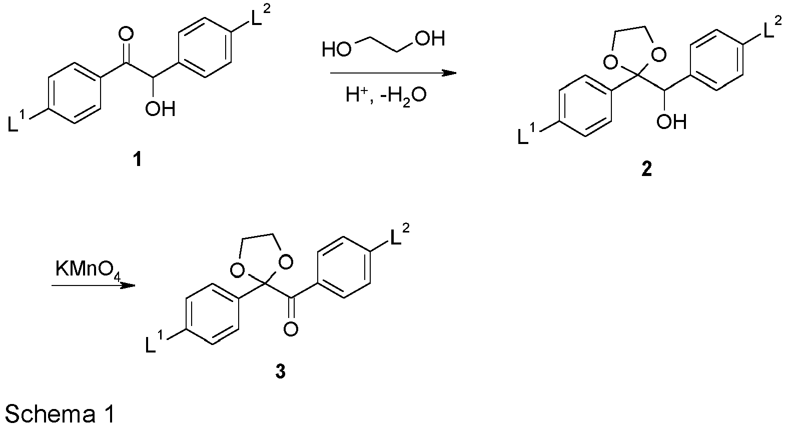

Ein Zugang zu Dimethylketalen (**5**) unter basischen Bedingungen durch Umsetzung mit Methyliodid in Gegenwart von Silber- oder Bariumoxid in Dimethylformamid ist in R. Kuhn, H. Trischmann, Chem. Ber. 1961, 94, 2258-2263 beschrieben (Schema 2).

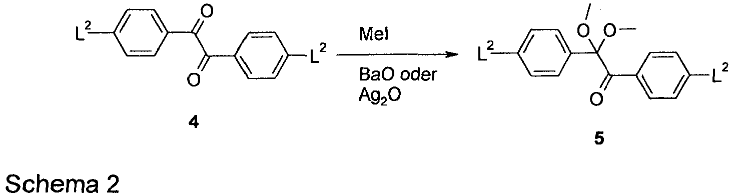

Ein weiterer Zugang zu Benzilmonoketalen ist durch Umsetzung von Benzil mit Alkoholen in Gegenwart von Thionylchlorid gegeben, wie in Schema 3 gezeigt. Schema 3 zeigt auch eine beispielhafte Abwandlung eines bevorzugten, kommerziell erhältlichen Ausgangsmaterials (Dihydroxybenzil, 6) zur Synthese der erfindungsgemäßen Verbindungen.

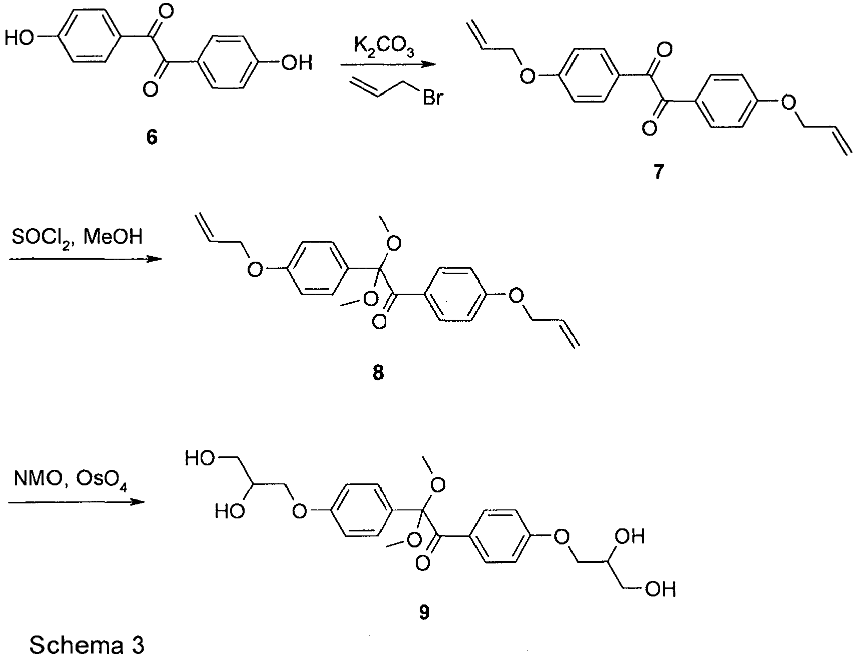

Das als Vorstufe für die erfindungsgemäß verwendeten Aminderivate geeignete Diaminobenzil (10) ist in US 2005266255 offengelegt. Dieses kann beispielsweise nach M. F. Sorokin, Izvestiya Vysshikh Uchebnykh Zavedenii, Khimiya i Khimicheskaya Tekhnologiya 1982, 25, 355-360 mit Glycidol zur Verbindung 11 umgesetzt werden (Schema 4).

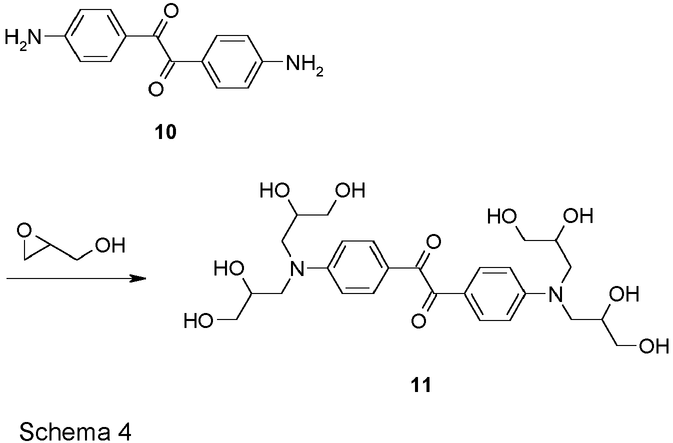

Ebenfalls Gegenstand der Erfindung sind Flüssigkristallanzeigen, die unter Verwendung der erfindungsgemäßen Dichtmittel hergestellt worden sind. In einer bevorzugten Ausführungsform werden die erfindungsgemäßen Flüssigkristallanzeigen im ODF-Verfahren hergestellt.
Die erfindungsgemäßen Flüssigkristallanzeigen sind bevorzugt PS-VA, PS-IPS, PS-FFS, PS-OCB oder PS-TN-Anzeigen.

### Beispiele

Die vorliegende Erfindung wird durch die nachfolgenden, nicht einschränkenden Beispiele detailliert beschrieben.

### Synthese von 1,2-bis[4-(2,3-dihydroxypropoxy)phenyl]-2,2-dimethoxy-ethanon

### 1.1 4,4'-Bis(allyloxy)benzil

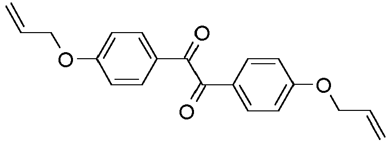

Zu einer Suspension von 4,4'-Dihydroxybenzil (16.8 g, 69.4 mmol) und Kaliumcarbonat (21,1 g, 153 mmol) in 250 ml Dimethylformamid wird eine Lösung von Allylbromid (23,0 g, 190 mmol) in 50 ml Dimethylformamid zutropfen gelassen. Der Ansatz wird über Nacht bei 80 °C rühren gelassen, auf Wasser gegeben und mit Dichlormethan extrahiert. Die vereinigten org. Phasen werden mit Wasser gewaschen, über Natriumsulfat getrocknet und i. Vak. eingeengt. Der Rückstand wird mit Dichlormethan an Kieselgel chromatographiert. Man erhält 4,4'-Bis(allyloxy)benzil als gelblichen Feststoff.

### 1.2 1,2-bis(4-Allyloxyphenyl)-2,2-dimethoxy-ethanon

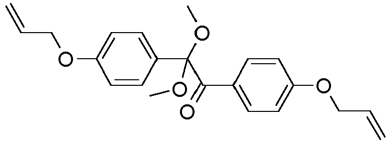

Zu einer Suspension von 4,4'-Bis(allyloxy)benzil (17,7 g, 55,0 mmol) und Thionylchlorid (53.0 ml, 0,740 mol) werden 60 ml Methanol langsam so wird der Ansatz 6 h auf 50°C erhitzt, vorsichtig durch Zugabe von verd. Natronlauge neutralisiert und mit Essigester extrahiert. Die vereinigten org. Phasen werden mit Wasser gewaschen und über Natriumsulfat getrocknet. Das Lösungsmittel wird i. Vak. entfernt und der Rückstand durch Chromatographie mit Dichlormethan/Essigester (9:1) an Kieselgel gereinigt. Man erhält 1,2-Bis(4-allyloxyphenyl)-2,2-dimethoxy-ethanon als gelbes Öl.

### 1.3 1,2-Bis[4-(2,3-dihydroxypropoxy)phenyl]-2,2-dimethoxy-ethanon

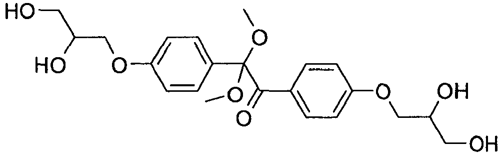

Zu einer Lösung von 1,2-Bis(4-allyloxyphenyl)-2,2-dimethoxy-ethanon (7,6 g) und 4-Methylmorpholin-N-oxid (8,2 g) in 90 ml Aceton und 20 ml Wasser werden tropfenweise 6.4 ml einer 4proz. wäßr. Osmiumtetroxidlösung so zugegeben, daß die Temperatur 20°C nicht übersteigt. Nach dem Rühren über Nacht werden 0,6g Natriummetabisulfit hinzugefügt und der Ansatz 1 h rühren gelassen. Die Lösung wird mit Essigester extrahiert und die vereinigten org. Phasen durch Chromatographie mit Dichlormethan/Methanol (9:1) an Kieselgel gereinigt. Man erhält 1,2-Bis[4-(2,3-dihydroxypropoxy)phenyl]-2,2-dimethoxy-ethanon als wachsartigen Feststoff.
¹H-NMR (*d*₆-DMSO, 500 MHz, mit TFA-Austausch)
δ = 7.79 ppm (d., 4 H, Ar-H), 7.79 (d., 4 H, Ar-H), 4.06 -3.82 (br. m., 8 H, 4xCH₂O), 3.45 (br. m., 2 H, 2xCH₂CHOHCH₂), 3.14 (s., 6 H, 2xOCH₃).

### Anwendungsbeispiel

Eine nematische Flüssigkristallmischung M-1 wird wie folgt hergestellt:

| Mischung M-1 | | |
|---|---|---|
| Zusammensetzung | | |
| Verbindung | | Konzentration /Massen-% |
| Nr. | Strukturformel | |
| 1 | 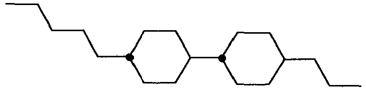 | 9,50 |
| 2 | 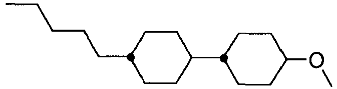 | 5,00 |
| 3 | 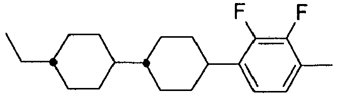 | 9,50 |
| 4 | 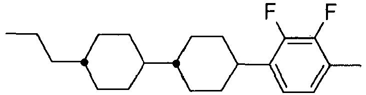 | 10,50 |
| 5 | 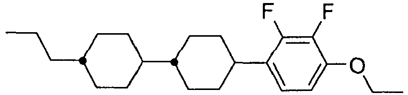 | 10,50 |
| 6 | 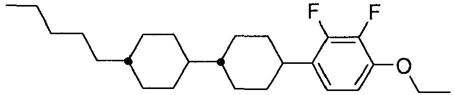 | 9,50 |
| 7 | 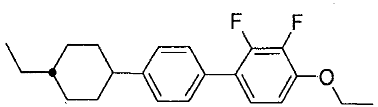 | 12,00 |
| 8 | 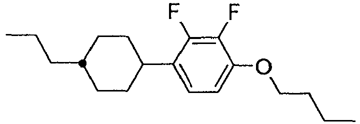 | 9,00 |
| 9 | 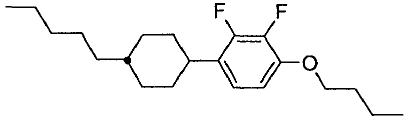 | 11,00 |
| 10 | 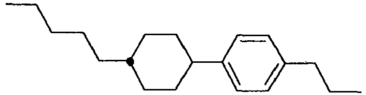 | 13,50 |
| ∑ | | 100,00 |

Eine polymerisierbare Flüssigkristallmischung P-1 wird hergestellt bestehend aus
1. 99,7 Gew.% der nematische Flüssigkristallmischung M-1 und
2. 0,3 Gew.% der polymerisierbaren Verbindung RM-1

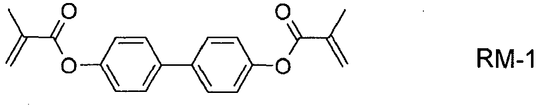

Drei Proben von je 0,5 g der Mischung P-1 werden zusammen mit je1 g des kommerziell erhältlichen Dichtmittels Unocol 2094® (APM Technica GmbH, Pürgen-Ummendorf), letztere jeweils enthaltend
1. keinen Photoinitiator
2. 3,0 Gew.-% des erfindungsgemäßen Photoinitiators aus Beispiel 1
3. 3,0 Gew.-% des kommerziell erhältlichen Photoinitiators Irgacure651® der unten gezeigten Struktur
in einem dicht verschlossenen Probenglas unter Ausschluß von Luft und Licht 7d bei 80°C gelagert. Eine vierte Probe der Mischung P-1 wird ohne die Gegenwart von Dichtmittel unter denselben Bedingungen gelagert (Blindversuch).

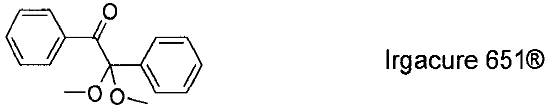

**Tabelle 2**

| Bsp. | Mischung | Dichtmittel | Photoinitiator | Initiatorkonzentration |
|---|---|---|---|---|
| 0.1 (Blindversuch) | P-1 | -- | -- | -- |
| 0.2 (Vergleich) | P-1 | Unocol 2094® | -- | -- |
| 1 | P-1 | Unocol 2094® | Irgacure651® | 3,0 Gew.% |
| 2 | P-1 | Unocol 2094® | Bsp. 1 | 3,0 Gew.% |

Anschließend werden die Mischungen vom Dichtmittel abgetrennt, in jeweils zwei Teile geteilt, in PS-VA-Testzellen gefüllt und bei 40 °C für die Dauer von 60 s bzw. 180 s mit einer Metallhalogenid-UV-Lampe mit einer Intensität von 100 mW/ cm² bestrahlt.

Die PS-VA-Testzellen bestehen aus zwei planparallelen Glasplatten mit einem Zellspalt von 4µm, die jeweils mit einer ITO-Elektrode und darauf mit einer Polyimid-Orientierungsschicht beschichtet sind.

Nach der Bestrahlung wird die Konzentration der polymerisierbaren Verbindung RM-1 durch HPLC bestimmt. Die Ergebnisse sind in Tabelle 3 aufgeführt.

**Tabelle 3**

| Bsp. | Konzentration RM /rel % nach | | |
|---|---|---|---|
| | t = 0 s | t = 60 s | t = 180 s |
| 0.1 (Blindversuch) | 100 | 88 | 56 |
| 0.2 (Vergleich) | 70 | 64 | 51 |
| 1 | 71 | 66 | 51 |
| 2 | 72 | 0 | 0 |

Wie aus Tabelle 3 ersichtlich ist, ist nach dem Lagertest in den Proben aus den Beispielen 0.2, 1 und 2 etwa gleich viel der polymerisierbaren Verbindung RM-1 nachweisbar (70-72%). Im Blindversuch (Bsp. 0.1, ohne Dichtmittel) ist etwas mehr RM-1 nachweisbar (relativ 100%), was auf ein Herauslösen des RM aus dem Flüssigkristall durch das Dichtmittel oder eine langsame Zersetzung des RM-1 während des Lagertests durch Komponenten des Dichtmittels in den anderen drei Proben hindeutet. Wie weiterhin ersichtlich ist, führt die Gegenwart von Irgacure651® während des Lagertests anschließend zu einer raschen Polymerisation von RM-1 bei UV-Bestrahlung der Flüssigkristallmischung; nach 60 s ist bereits kein RM-1 mehr nachweisbar, was dafür spricht, daß sich erhebliche Mengen an Photoinitiator in der Flüssigkristallmischung gelöst haben (Bsp. 2).

Die Abnahme der RM-Konzentration in den Beispielen 0.1 und 0.2 beruht auf der, wenngleich geringen, Reaktivität des RM-1, das auch ohne Photoinitiator langsam polymerisiert.
Wie man sieht, ist die Abnahme der Konzentration an RM-1 nach Kontakt der Mischung P-1 mit dem erfindungsgemäßen Dichtmittel enthaltend den Photoinitiator aus Beispiel 1 vergleichbar mit der Probe aus Beispiel 0.2 (ohne Photoinitiator), was dafür spricht, daß während des Lagertests kein erfindungsgemäßer Photoinitiator aus dem Dichtmittel im Flüssigkristall gelöst wurde, weshalb der erfindungsgemäße Photoinitiator hervorragend zur Herstellung von Flüssigkristallanzeigen geeignet ist, insbesondere, wenn unerwünschte Reaktionen während des Härtens von Dichtmitteln bei der Herstellung vermieden werden sollen.

## Patentansprüche

1. Verwendung von Verbindungen der Formel I als Photopolymerisationsinitiatoren von polymerisierbaren Stoffgemischen, die ungesättigte Verbindungen enthalten, oder zur photochemischen Vernetzung von linearen Polymeren, in Dichtmitteln für Flüssigkristallanzeigen 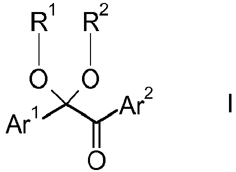 worin
R¹, R² unabhängig voneinander ein geradkettiger oder verzweigter oder cyclischer Alkyl-, Cycloalkylalkyl-, Arylalkyl, Alkenyl- oder Cycloalkylalkenylrest mit bis zu 20 C-Atomen oder ein Arylalkenylrest mit 8 bis 20 C-Atomen, in denen jeweils ein oder mehrere CH₂-Gruppen durch -CO- , -O- und/oder -S-so ersetzt sein können, daß keine O- oder S-Atome benachbart sind und in denen ein oder mehrere Wasserstoffatome durch Halogen ersetzt sein können, oder beide Reste R¹ und R² gemeinsam ein zweibindiges Brückenglied W,
W ein zweiwertiger Rest eines aliphatischen, geradkettigen oder verzweigten Diols mit 2 bis 10 C-Atomen,
Ar¹, Ar² unabhängig voneinander jeweils durch einen oder mehrere Substituenten L substituierte Arylreste, die zusätzlich durch Halogen, Alkyl, Alkoxy mit jeweils 1 bis 5 C-Atomen, oder Phenyl substituiert sein können,
L bei jedem Auftreten gleich oder verschieden ein hydrophiler Rest
bedeuten.

2. Verwendung nach Anspruch 1, **dadurch gekennzeichnet, daß** in Formel I
R¹, R² unabhängig voneinander ein geradkettiger oder verzweigter Alkylrest mit 1 bis 7 C-Atomen,
Ar¹, Ar² unabhängig voneinander jeweils durch einen Substituenten
L substituiertes 1,4-Phenylen,
L -CH₂-L', -O-L' oder -N(L')₂
L' ein geradkettiger oder verzweigter Alkylrest mit 1 bis 19 C-Atomen, in dem eine oder mehrere CH₂-Gruppen durch Cycloalkandiylreste mit 3 bis 8 Ringatomen ersetzt sein können und in dem eine oder mehrere nicht benachbarte CH₂-Gruppen durch O ersetzt sein können und in dem ein oder mehrere H-Atome durch -OH oder 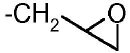 ersetzt sind,
bedeuten.

3. Verwendung nach Anspruch 1, **dadurch gekennzeichnet, daß** in Formel I
R¹, R² W,
W ein zweiwertiger Rest eines aliphatischen, geradkettigen oder verzweigten Diols mit 2 bis 10 C-Atomen,
Ar¹, Ar² unabhängig voneinander jeweils durch einen Substituenten L substituiertes 1,4-Phenylen,
L -CH₂-L', -O-L' oder -N(L')₂
L' ein geradkettiger oder verzweigter Alkylrest mit 1 bis 19 C-Atomen, in dem eine oder mehrere CH₂-Gruppen durch Cycloalkandiylreste mit 3 bis 8 Ringatomen ersetzt sein können und in dem eine oder mehrere nicht benachbarte CH₂-Gruppen durch O ersetzt sein können und in dem ein oder mehrere H-Atome durch -OH oder 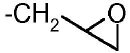 ersetzt sind,
bedeuten.

4. Verwendung nach einem oder mehreren der Ansprüche 1 bis 3, **dadurch gekennzeichnet, daß** die Verbindungen der Formel I ausgewählt sind aus der Gruppe der Verbindungen der folgenden Unterformeln: 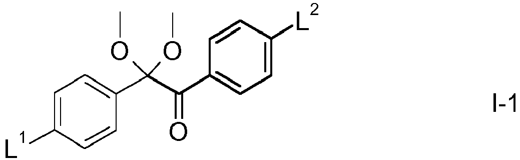 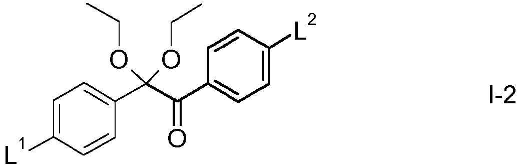 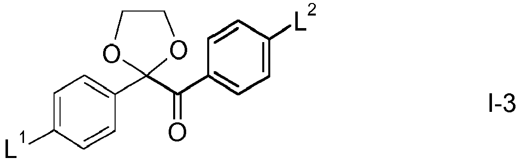 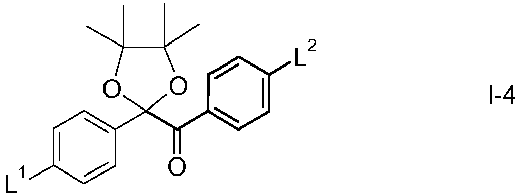 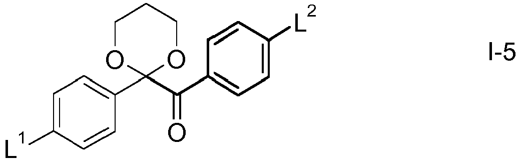 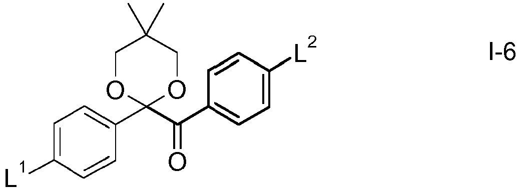 wobei L¹ und L² unabhängig voneinander die in Anspruch 2 und 3 für L angegebene Bedeutung haben.

5. Verwendung nach Anspruch 1, **dadurch gekennzeichnet, daß**
L -(CH₂)ₚOG,-O(CH₂)ₘ₊₁OG, -(CH₂)ₙ(OCH₂CH₂)ₘOG, -(O)ₙ(CH₂)ₘCH(OH)C H₂OG, -O(CH₂CH₂)ₘ₊₁OCH₂CH(OG)CH₂OG, -OC(CH₂OG)₃,-OC(CH₂OG)₂(CH₂)ₙH, -(CH₂)ₙOCH₂CH(OH)CH₂OG, -(OCH₂C H₂)ₘOCH₂CH(OG)CH₂OG, -(CH₂)ₙOCH₂(CH)ₘ((CH₂)ₘOG)₂, -( CH₂)ₙOC(CH₂OG)₃, --(CH₂)ₘOC(CH₂OG)₂(CH₂)ₙH,-N((CH₂)ₘ₊₁OG)₂, -N((CH₂CH₂O)ₘG)₂, -N((CH₂)ₘCH(OG)CH₂O G)₂ oder 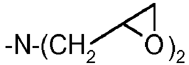
m eine ganze Zahl von 1 bis 10,
n, p eine ganze Zahl von 0 bis 10
G 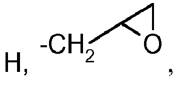 oder
ein Monosaccharidrest ausgewählt aus Glycopyranose und Glycofuranose,
mit der Maßgabe, daß wenn p gleich 0 ist G nicht H sein kann,
bedeutet.

6. Verwendung nach einem oder mehreren der Ansprüche 1 bis 5, **dadurch gekennzeichnet, daß** die Verbindungen der Formel I einen Octan-1-ol-Wasser-Verteilungskoeffizienten von logP ≤ 3.2 aufweisen.

7. Dichtmittel für Flüssigkristallanzeigen, **dadurch gekennzeichnet, daß** es eine oder mehrere der in den Ansprüchen 1 bis 6 aufgeführten Verbindungen enthält.

8. Dichtmittel nach Anspruch 7, **dadurch gekennzeichnet, daß** es photochemisch aushärtbar ist.

9. Dichtmittel nach Anspruch 8, **dadurch gekennzeichnet, daß** es zusätzlich thermisch aushärtbar ist.

10. Flüssigkristallanzeige, **dadurch gekennzeichnet, daß** sie unter Verwendung eines Dichtmittels nach einem oder mehreren der Ansprüche 7 bis 9 hergestellt worden ist.

11. Flüssigkristallanzeige nach Anspruch 10, **dadurch gekennzeichnet, daß** sie im ODF-Verfahren hergestellt worden ist.

12. Flüssigkristallanzeige nach Anspruch 10 oder 11, **dadurch gekennzeichnet, daß** es sich um eine PS-VA, PS-IPS, PS-FFS, PS-OCB oder PS-TN-Anzeige handelt.

## Claims

1. Use of compounds of the formula I as photopolymerisation initiators of polymerisable substance mixtures which comprise unsaturated compounds, or for the photochemical crosslinking of linear polymers, in sealants for liquid-crystal displays, 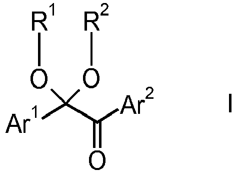 in which
R¹, R², independently of one another, denote a straight-chain or branched or cyclic alkyl, cycloalkylalkyl, arylalkyl, alkenyl or cycloalkylalkenyl radical having up to 20 C atoms or an arylalkenyl radical having 8 to 20 C atoms, in each of which one or more CH₂ groups may be replaced by -CO-, -O- and/or -S- in such a way that no O or S atoms are adjacent and in which one or more hydrogen atoms may be replaced by halogen, or both radicals R¹ and R² together denote a divalent bridging member W,
W denotes a divalent radical of an aliphatic, straight-chain or branched diol having 2 to 20 C atoms,
Ar¹, Ar², independently of one another, denote aryl radicals which are in each case substituted by one or more substituents L and which may additionally be substituted by halogen, alkyl, alkoxy, each having 1 to 5 C atoms, or phenyl,
L on each occurrence, identically or differently, denotes a hydrophilic radical.

2. Use according to Claim 1, **characterised in that**, in formula I,
R¹, R², independently of one another, denote a straight-chain or branched alkyl radical having 1 to 7 C atoms,
Ar¹, Ar², independently of one another, denote 1,4-phenylene, which is in each case substituted by a substituent L,
L denotes -CH₂-L', -O-L' or -N(L')₂,
L' denotes a straight-chain or branched alkyl radical having 1 to 19 C atoms, in which one or more CH₂ groups may be replaced by cycloalkanediyl radicals having 3 to 8 ring atoms and in which one or more non-adjacent CH₂ groups may be replaced by O and in which one or more H atoms may be replaced by -OH or

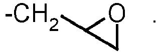

3. Use according to Claim 1, **characterised in that**, in formula I,
R¹, R² denote W,
W denotes a divalent radical of an aliphatic, straight-chain or branched diol having 2 to 10 C atoms,
Ar¹, Ar², independently of one another, denote 1,4-phenylene, which is in each case substituted by a substituent L,
L denotes -CH₂-L', -O-L' or -N(L')₂,
L' denotes a straight-chain or branched alkyl radical having 1 to 19 C atoms, in which one or more CH₂ groups may be replaced by cycloalkanediyl radicals having 3 to 8 ring atoms and in which one or more non-adjacent CH₂ groups may be replaced by O and in which one or more H atoms may be replaced by -OH or

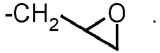

4. Use according to one or more of Claims 1 to 3, **characterised in that** the compounds of the formula I are selected from the group of the compounds of the following sub-formulae: 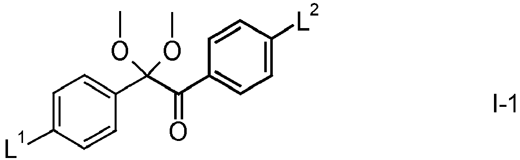 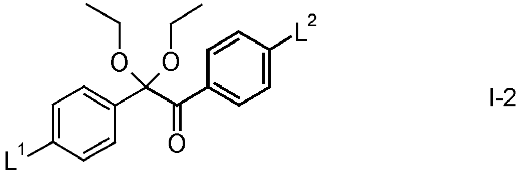 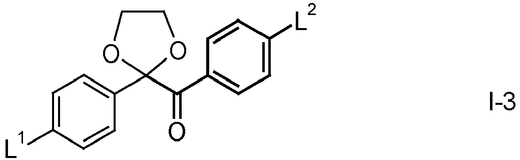 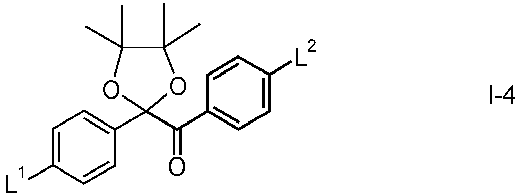 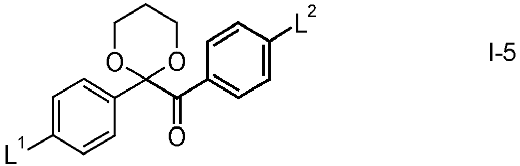 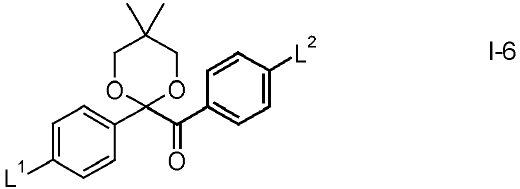 where L¹ and L², independently of one another, have the meaning indicated for L in Claims 2 and 3.

5. Use according to Claim 1, **characterised in that**
L denotes -(CH₂)ₚOG, -O(CH₂)ₘ₊₁OG, -(CH₂)ₙ(OCH₂CH₂)ₘOG, -(O)ₙ(CH₂)ₘCH(OH)CH₂OG, -O(CH₂CH₂)ₘ₊₁OCH₂CH(OG)CH₂-OG, -OC(CH₂OG)₃, -OC(CH₂OG)₂(CH₂)ₙH, -(CH₂)ₙOCH₂CH(OH)CH₂OG, -(OCH₂CH₂)ₘOCH₂CH(OG)CH₂OG, -(CH2)nOCH2(CH)m((CH2)mOG)2, -(CH₂)ₙOC(CH₂OG)₃, -(CH₂)ₘOC(CH₂OG)₂(CH₂)ₙH, -N((CH₂)ₘ₊₁OG)₂, -N((CH₂CH₂O)ₘG)₂, -N((CH₂)ₘCH(OG)CH₂OG)₂ or 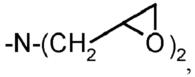
m denotes an integer from 1 to 10,
n, p denote an integer from 0 to 10,
G denotes H, 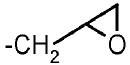 or
a monosaccharide radical selected from glucopyranose and glucofuranose,
with the proviso that G cannot be H if p is equal to 0.

6. Use according to one or more of Claims 1 to 5, **characterised in that** the compounds of the formula I have an octan-1-ol/water partition coefficient of logP ≤ 3.2.

7. Sealant for liquid-crystal displays, **characterised in that** it comprises one or more of the compounds mentioned in Claims 1 to 6.

8. Sealant according to Claim 7, **characterised in that** it is photochemically curable.

9. Sealant according to Claim 8, **characterised in that** it is additionally thermally curable.

10. Liquid-crystal display, **characterised in that** it has been produced using a sealant according to one or more of Claims 7 to 9.

11. Liquid-crystal display according to Claim 10, **characterised in that** it has been produced by the ODF process.

12. Liquid-crystal display according to Claim 10 or 11, **characterised in that** it is a PS-VA, PS-IPS, PS-FFS, PS-OCB or PS-TN display.

## Revendications

1. Utilisation de composés de la formule I en tant qu'initiateurs de photo-polymérisation de mélanges de substances polymérisables, lesquels comprennent des composés non saturés, ou pour la réticulation photochimique de polymères linéaires, dans des agents de scellement et d'étanchéité pour les affichages à cristaux liquides, 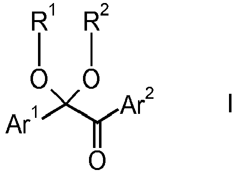 dans laquelle
R¹, R² représentent, de manière indépendante l'un de l'autre, un radical alkyle, cycloalkylalkyle, arylalkyle, alkényle ou cycloalkylalkényle en chaîne droite ou ramifié ou cyclique qui comporte jusqu'à 20 atomes de C ou un radical aryl-alkényle qui comporte de 8 à 20 atomes de C, et dans chacun d'entre eux, un ou plusieurs groupe(s) CH₂ peut/ peuvent être remplacé(s) par -CO-, -O- et/ou -S- de telle sorte que ni des atomes de O, ni des atomes de S ne soient adjacents et où un ou plusieurs atome(s) d'hydrogène peut/peuvent être remplacé(s) par halogène, ou les deux radicaux R¹ et R² représentent en association un élément de pontage divalent W,
W représente un radical divalent d'un diol aliphatique, en chaîne droite ou ramifié qui comporte de 2 à 20 atomes de C,
Ar¹, Ar² représentent, de manière indépendante l'un de l'autre, des radicaux aryle qui sont dans chaque cas substitués par un ou plusieurs substituant(s) L et qui peuvent de façon additionnelle être substitués par halogène, alkyle, alcoxy, dont chacun comporte de 1 à 5 atome(s) de C, ou phényle,
L représente pour chaque occurrence, de manière identique ou différente, un radical hydrophile.

2. Utilisation selon la revendication 1, **caractérisée en ce que**, dans la formule I,
R¹, R² représentent, de manière indépendante l'un de l'autre, un radical alkyle en chaîne droite ou ramifié qui comporte de 1 à 7 atome(s) de C,
Ar¹, Ar² représentent, de manière indépendante l'un de l'autre, 1,4-phénylène, lequel est dans chaque cas substitué par un substituant L,
L représente -CH₂-L', -O-L' ou -N(L')₂,
L' représente un radical alkyle en chaîne droite ou ramifié qui comporte de 1 à 19 atome(s) de C, où un ou plusieurs groupe(s) CH₂ peut/peuvent être remplacé(s) par des radicaux cycloalkanediyle qui comportent de 3 à 8 atomes de cycle et où un ou plusieurs groupe(s) CH₂ non adjacents peut/peuvent être remplacé(s) par O et où un ou plusieurs atome(s) de H peut/peuvent être remplacé(s) par -OH ou par

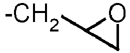

3. Utilisation selon la revendication 1, **caractérisée en ce que**, dans la formule I,
R¹, R² représente W,
W représente un radical divalent d'un diol aliphatique, en chaîne droite ou ramifié qui comporte de 2 à 10 atomes de C,
Ar¹, Ar² représentent, de manière indépendante l'un de l'autre, 1,4-phénylène, lequel est dans chaque cas substitué par un substituent L,
L représente -CH₂-L', -O-L' ou -N(L')₂,
L' représente un radical alkyle en chaîne droite ou ramifié qui comporte de 1 à 19 atome(s) de C, où un ou plusieurs groupe(s) CH₂ peut/peuvent être remplacé(s) par des radicaux cycloalkanediyle qui comportent de 3 à 8 atomes de cycle et où un ou plusieurs groupe(s) CH₂ non adjacents peut/peuvent être remplacé(s) par O et où un ou plusieurs atome(s) de H peut/peuvent être remplacé(s) par -OH ou par

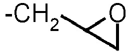

4. Utilisation selon une ou plusieurs des revendications 1 à 3, **caractérisée en ce que** les composés de la formule I sont sélectionnés parmi le groupe des composés des sous-formules qui suivent : 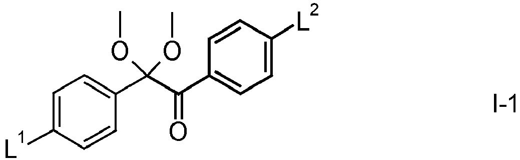 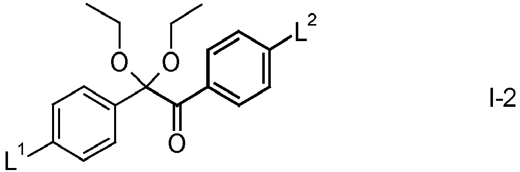 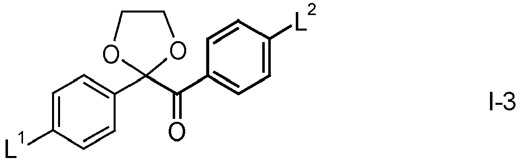 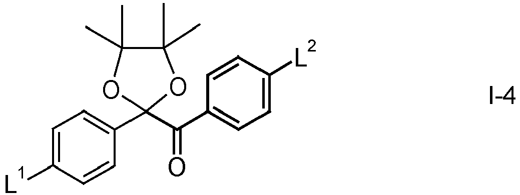 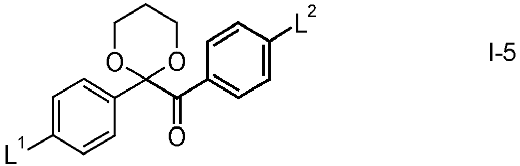 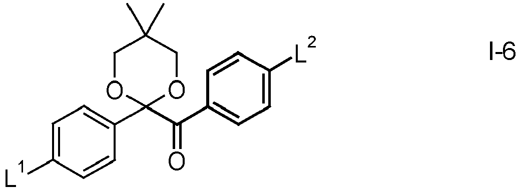 dans lesquelles L¹ et L² présentent, de manière indépendante l'un de l'autre, la signification qui a été indiquée pour L selon les revendications 2 et 3.

5. Utilisation selon la revendication 1, **caractérisée en ce que**
L représente -(CH₂)ₚOG, -O(CH₂)ₘ₊₁OG, -(CH₂)ₙ(OCH₂CH₂)ₘOG, -(O)ₙ(CH₂)ₘCH(OH)CH₂OG, -O(CH₂CH₂)ₘ₊₁OCH₂CH(OG)CH₂-OG, -OC(CH₂OG)₃, -OC(CH₂OG)₂(CH₂)ₙH, -(CH₂)ₙOCH₂CH(OH)CH₂OG, -(OCH₂CH₂)ₘOCH₂CH(OG)CH₂OG, -(CH₂)ₙOCH₂(CH)ₘ((CH₂)ₘOG)₂, -(CH₂)ₙOC(CH₂OG)₃, -(CH₂)ₘOC(CH₂OG)₂(CH₂)ₙH, -N((CH₂)ₘ₊₁OG)₂, -N((CH₂CH₂O)ₘG)₂, -N((CH₂)ₘCH(OG)CH₂OG)₂ ou 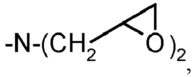
m représente un entier de 1 à 10,
n, p représentent un entier de 0 à 10,
G représente H, 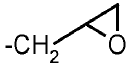 ou
un radical monosaccharide qui est sélectionné parmi le gluco-pyranose et le glucofuranose,
étant entendu que G ne peut pas être H si p est égal à 0.

6. Utilisation selon une ou plusieurs des revendications 1 à 5, **caractérisée en ce que** les composés de la formule I présentent un coefficient de partition octan-1-ol/eau qui est tel que logP ≤ 3,2.

7. Agent de scellement et d'étanchéité pour les affichages à cristaux liquides, **caractérisé en ce qu'**il comprend un ou plusieurs des composés qui ont été mentionnés selon les revendications 1 à 6.

8. Agent de scellement et d'étanchéité selon la revendication 7, **caractérisé en ce qu'**il peut être durci photochimiquement.

9. Agent de scellement et d'étanchéité selon la revendication 8, **caractérisé en ce que**, de façon additionnelle, il peut être durci thermiquement.

10. Affichage à cristaux liquides, **caractérisé en ce qu'**il a été fabriqué en utilisant un agent de scellement et d'étanchéité selon une ou plusieurs des revendications 7 à 9.

11. Affichage à cristaux liquides selon la revendication 10, **caractérisé en ce qu'**il a été fabriqué au moyen du procédé ODF.

12. Affichage à cristaux liquides selon la revendication 10 ou 11, **caractérisé en ce qu'**il s'agit d'un affichage PS-VA, PS-IPS, PS-FFS, PS-OCB ou PS-TN.
